# EUROPÄISCHE PATENTSCHRIFT

(11) **EP 1 697 330 B1**
(45) Veröffentlichungstag und Bekanntmachung des Hinweises auf die Patenterteilung: **20.11.2013**
(21) Anmeldenummer: 04803569.5
(22) Anmeldetag: 07.12.2004
(51) Int. Cl.: C07D 233/60

(54) **VERFAHREN ZUR HERSTELLUNG VON N,N'-CARBONYLDIAZOLEN**
METHOD FOR THE PRODUCTION OF N,N -CARBONYLDIAZOLES
PROCÉDÉ DE PRODUCTION DE N,N'-CARBONYLDIAZOLES

(30) Priorität: 19.12.2003 DE 10359797
(43) Veröffentlichungstag der Anmeldung: 06.09.2006
(73) Patentinhaber: Saltigo GmbH, 51369 Leverkusen (DE)
(72) Erfinder: JOB, Andreas, 50858 Köln (DE); GRIEHSEL, Bernd, 46242 Bottrop (DE); SCHERER, Johannes, 51375 Leverkusen (DE)
(74) Vertreter: Wichmann, Birgid
(86) Internationale Anmeldenummer: PCT/EP2004/013876
(87) Internationale Veröffentlichungsnummer: WO 2005/063718

(56) Entgegenhaltungen:
- WO-A-00/06551
- DE-B- 1 033 210

## Beschreibung

Die vorliegende Erfindung betrifft ein verbessertes Verfahren zur Herstellung von N,N'-Carbonyldiazolen durch Umsetzung von Azolen mit Phosgen.

Es ist bereits bekannt, dass man N,N'-Carbonyldiazole erhalten kann, indem man Azole mit Phosgen umsetzt (siehe DE-AS 10 33 210 und Liebigs Ann. Chem. 1957, 609, 75). Dabei werden Tetrahydrofuran, andere Ether und aliphatische oder aromatische Kohlenwasserstoffe als mögliche Lösungsmittel beschrieben, insbesondere wird wasserfreies Tetrahydrofuran für die Reaktion verwendet. Hierbei wird eine Lösung des gesamten Azols in dem jeweiligen Lösungsmittel vorgelegt und dann das Phosgen eingeleitet. Die Umsetzung erfolgt bei Raumtemperatur. Auffällig ist die geringe Konzentration des Azols in THF als Lösungsmittel von 2 bis 4 Gew.-%. In einem entsprechenden, aus Chem. Ber. 1963, 96, 3374 bekannten Verfahren werden unter Verwendung von THF-Benzol-Gemischen als Lösungsmittel Konzentrationen von etwa 7 Gew.% erreicht.

Nach einem neueren Verfahren gemäß EP-A-692 476 können in aromatischen Lösungsmitteln wie Benzol, Toluol, Xylolen, Chlorbenzolen oder Gemischen davon, die vor der Umsetzung jeweils durch Andestillieren entwässert werden, bei Temperaturen von 50 bis 120°C etwas höhere Konzentrationen des Azols im Lösungsmittel erreicht werden. Beschrieben sind Konzentrationen im Bereich bis zu 12 Gew.-%. Hierbei wird zunächst das Lösungsmittel durch Andestillieren entwässert, dann das Azol zugegeben, dieses unter Erwärmung gelöst und dann Phosgen eingeleitet.

In der WO-A-00/14072 ist ferner ein Verfahren zur Herstellung von Carbonyldiimidazol aus Imidazol und Phosgen bei einer Temperatur von 60 bis 80°C beschrieben, welches in ortho-, meta- oder para-Xylol, Mischungen daraus oder in Chlorbenzol als Lösungsmittel durchgeführt wird und bei dem das als Koppelprodukt anfallende Imidazol-Hydrochlorid als Schmelze durch Phasentrennung aus der erhaltenen Reaktionsmischung bei einer Temperatur größer 100°C abgetrennt wird. Die Umsetzung als solche wird durchgeführt, indem man Phosgen zu dem in Lösung vorgelegten Imidazol zudosiert.

Aus der DE-A-198 33 913 ist ein Verfahren zur Herstellung von N,N'-Carbonyldiazolen bekannt, bei dem unter Verwendung aromatischer Lösungsmittel wie Benzol, Toluol, Xylol oder chlorierten Benzolen gearbeitet wird, die vorab durch Andestillieren entwässert werden. Wesentlich ist, dass das Azol, gelöst in einem der genannten aromatischen Lösungsmittel, und Phosgen paraiiei in weiteres vorgelegtes Lösungsmittel zudosiert werden. Durch diese Art der Verfahrensführung ist es möglich, eine Konzentration des Azols von bis zu 33 Gew.% zu erreichen. Da aber ein Teil des verwendeten Lösungsmittels mit den Reaktanden zugegeben wird, entfällt hier die Möglichkeit, das gesamte Lösungsmittelvolumen in dem, für die Reaktion vorgesehenen Kessel azeotrop zu trocknen.

Sowohl bei dem Verfahren gemäß DE-A-198 33 913 als auch den zuvor beschriebenen Verfahren, die aromatische Lösungsmittel verwenden und die bei Temperaturen von über 50°C ablaufen, besteht die Gefahr, dass der bei der Reaktion entstehende Azol-Hydrochlorid-Niederschlag als zähe, klebrige Masse anfällt. Diese haftet fest an Gefäßwand und Rührer, wodurch die Rührbarkeit des Systems sehr erschwert wird. Die Schwergängigkeit des Rührens begrenzt dadurch die maximal mögliche Raum-Zeit-Ausbeute auf sehr geringe Werte. Bei der Verfestigung des Niederschlags gegen Ende der Phosgenzugabe entstehen harte Kugeln, die zudem am Reaktionsgefäß und seinen Einbauten (z.B. Rührer, Tauchrohre etc.) Beschädigungen verursachen können.

Es besteht deshalb das Bedürfnis nach einem verbesserten Verfahren zur Herstellung von N,N'-Carbonyldiazolen, bei dem derartige, klebrige und Probleme verursachende Azol-Hydrochlorid-Niederschläge nicht auftreten und ferner keine hohen Reaktionstemperaturen erforderlich sind, welche die Wirtschaftlichkeit des Verfahrens durch hohe Energiekosten verringern würden.

Gegenstand der Erfindung ist ein Verfahren zur Herstellung von N,N'-Carbonyldiazolen der allgemeinen Formel (I) 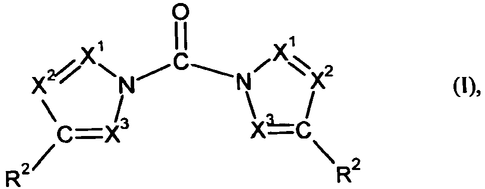 wobei entweder
- X¹ und X³: unabhängig voneinander jeweils für CR¹ stehen, wobei R¹ Wasserstoff oder geradkettiges oder verzweigtes C₁-C₆-Alkyl bedeutet und
- X²: für Stickstoff steht und
- R²: Wasserstoff bedeutet,
oder
- X¹ und X³: für CR¹ stehen, wobei der in X¹ befindliche Rest R¹ Wasserstoff oder geradkettiges oder verzweigtes C₁-C₆-Alkyl bedeutet und der in X³ befindliche Rest R¹ gemeinsam mit R² eine -CH=CH-CH=CH-Brücke bildet, und
- X²: für Stickstoff steht,
durch Umsetzung von Azolen der allgemeinen Formel **(II),** 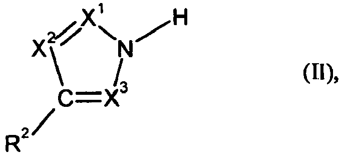 in der die verwendeten Reste und Symbole die für die allgemeine Formel (I) angegebenen Bedeutungen haben,
mit Phosgen in einem polaren Lösungsmittel, wobei dieses Verfahren dadurch gekennzeichnet ist, dass
(i) ein polares Lösungsmittel aus der Gruppe bestehend aus Ethern, Ketonen und chlorierten aliphatischen Lösungsmitteln verwendet wird, welches einen maximalen Wassergehalt von 0,5 Gew.-% besitzt, und
(ii) dass man das Azol der allgemeinen Formel (II) sowie das Phosgen in dieses Lösungsmittel so eindosiert, dass in der Zeit, in der 1 mol Azol der allgemeinen Formel (11) eindosiert wird, gleichzeitig 0,17 bis 0,34 mol Phosgen eindosiert werden, wobei die Reaktandenkonzentration (Azolkonzentration) bei über 40 Gew.% liegt.

Beim erfindungsgemäßen Verfahren entsteht und verbleibt das bei der Reaktion ausfallende Azol-Hydrochlorid, wie z.B. das Imidazolhydrochlorid, im Gegensatz zu dem in der DE-A-198 33 913 beschriebenen Verfahren immer als gut rührbarer, kristalliner Niederschlag, der keine Anbackungen und Verklebungen an Rührer oder Gefäßwand bildet. Durch den dispersen Habitus des Niederschlags ist der Rührwiderstand deutlich geringer als bei nicht erfindungsgemäßer Arbeitsweise. Man kann deshalb erfindungsgemäß deutlich höhere Reaktanden-Konzentrationen als bisher anwenden, was eine deutlich verbesserte Raum-Zeit-Ausbeute gegenüber dem Stand der Technik zur Folge hat. Im Verfahren der DE-A-198 33 913 wird eine Reaktandenkonzentration von 28 bis 33 Gew.% angegeben, während man mit dem erfindungsgemäßen Verfahren Reaktandenkonzentrationen von über 40 Gew.% und damit erheblich verbesserte Raum-Zeit-Ausbeuten erhält. Auch können Beschädigungen des Reaktors und seiner Einbauten durch harte Azol-Hydrochlorid-Konglomerate ausgeschlossen werden.

Darüber hinaus zeigt das erfindungsgemäße Verfahren eine sehr geringe Empfindlichkeit gegenüber einer Überphosgenierung, die bei anderen Verfahren zu Verfärbungen des isolierten Produkts führt.

Im erfindungsgemäßen Verfahren können entweder zwei verschiedene oder aber auch nur ein einziges Azol der allgemeinen Formel (II) eingesetzt werden. Im ersten Fall wird ein N,N'-Carbonyl-diazol der Formel (I) erhalten, bei dem die beiden Azolringe verschieden sind. Im zweiten Fall entsteht ein N,N'-Carbonyldiazol mit zwei identischen Azolringen. Diese zweite Durchführungsvariante ist die bevorzugte.

Es ist bevorzugt, dass X¹ für CH, X² für Stickstoff und X³ für CR¹ stehen, wobei R¹ und R² gemeinsam eine -CH=CH-CH=CH-Brücke bilden.

Besonders bevorzugt setzt man im erfindungsgemäßen Verfahren Imidazol oder Benzimidazol, als Azol der allgemeinen Formel (II) ein. Ganz besonders bevorzugt ist Imidazol.

Die genannten Azole der allgemeinen Formel (II) sind entweder käuflich erhältlich oder aber nach bekannten Verfahren des Standes der Technik herstellbar.

Phosgen kann in üblicher technischer Qualität eingesetzt werden. Es ist vorteilhaft, insgesamt pro mol Azol der allgemeinen Formel (II) 0,2 bis 0,3 mol, bevorzugt 0,22 bis 0,27 mol, insbesondere 0,24 bis 0,26 mol Phosgen, einzusetzen.

Das einzusetzende polare Lösungsmittel muss aus der Gruppe bestehend aus Ethern, Ketonen und chlorierten aliphatischen Lösungsmittel stammen.

Als Ether kommen beispielsweise lineare oder cyclische aliphatische Ether und Diether, insbesondere MTBE, Dimethylether, Diethylether, Dibutylether, THF, 2-Methyl-THF, 2,5-Dimethyl-THF, Dioxan, Ethylenglycoldimethylether oder Ethylenglycoldiethylether, aromatische Ether, insbesondere Anisol und chlorierte Derivate des Anisols sowie Gemische der vorgenannten Lösungsmittel zum Einsatz.

Als Ketone können beispielsweise lineare oder cyclische aliphatische Ketone, insbesondere Aceton, 2-Butanon, Diethylketon, Dipropylketon, Cyclopentanon, Cyclohexanon oder Cycloheptanon, sowie Gemische der vorgenannten Lösungsmittel eingesetzt werden.

Als chlorierte aliphatische Lösungsmittel können beispielsweise Methylenchlorid, Chloroform und 1,2-Dichlorethan eingesetzt werden.

Das eingesetzte polare Lösungsmittel aus der oben genannten Gruppe besitzt einen Wassergehalt von maximal 0,5 Gew.-%, bevorzugt von maximal 0,2 Gew.%, besonders bevorzugt von maximal 0,1 Gew.% und insbesondere von maximal 0,05 Gew.%. Lösungsmittel mit diesem Wassergehalt sind entweder käuflich oder aber durch entsprechendes Andestillieren/Trocknen erhältlich.

Das verwendete Azol der allgemeinen Formel (II) kann in Form einer Lösung oder einer Suspension in den vorgenannten Lösungsmitteln, wobei diese Lösung oder Suspension geeigneterweise eine Temperatur im Bereich von 20 bis 100°C, bevorzugt 40 bis 80°C besitzt, oder als Schmelze eindosiert werden. Durch diese Art der Dosierung wird die Kontrolle der Dosiergeschwindigkeit erleichtert.

Das erfindungsgemäße Verfahren wird üblicherweise bei einer Temperatur im Bereich von 20 bis 100°C, vorzugsweise bei 40 bis 80°C, insbesondere bei 40 bis 65°C durchgeführt.

Es ist ein wesentliches Merkmal des erfindungsgemäßen Verfahrens, dass man das Azol der allgemeinen Formel (II) sowie das Phosgen simultan zu dem polaren Lösungsmittel aus der Gruppe bestehend aus Ethern, Ketonen und chlorierten aliphatischen Lösungsmitteln so zudosiert, dass in der Zeit, in der I mol Azol der allgemeinen Formel (II) eindosiert wird, gleichzeitig 0,17 bis 0,34 mol, bevorzugt 0,2 bis 0,3 mol, insbesondere 0,24 bis 0,28 mol Phosgen eindosiert werden.

Hierbei kann so vorgegangen werden, dass im Reaktionsgefäß zunächst ausschließlich eine gewisse Menge des polaren Lösungsmittels vorgelegt wird und das Azol sowie Phosgen wie oben angegeben eindosiert werden.

In einer weiteren, bevorzugten Ausführungsform werden im Reaktionsgefäß bis zu 10 Gew.-%, bevorzugt 0,1 bis 2 Gew.% der Gesamtmenge des Azols in Form einer Lösung oder Suspension im Reaktionsgefäß vorgelegt und anschließend die weitere Menge des Azols und das Phosgen gleichzeitig wie angegeben zudosiert. Durch diese Vorgehensweise kann vermieden werden, dass zu Beginn der Reaktion Phosgen in einem deutlichen molaren Überschuss zum Azol (molares Verhältnis Phosgen:Azol = 0,3 oder höher) vorliegt. Solche Molverhältnisse begünstigen eine Zersetzung des Azols unter Dunkelfärbung des Reaktionsgemisches.

Im allgemeinen ist es vorteilhaft, nach dem gleichzeitigen Zudosieren der Reaktanden das Reaktionsgemisch noch einige Zeit im Bereich von 30 Minuten bis 5 Stunden bei gleicher Temperatur nachzurühren.

Zwecks Aufarbeitung schwemmt man das Reaktionsgemisch aus dem Reaktionsgefäß in eine Filtriervorrichtung aus. Da das Azol-Hydrochlorid auch nach Ende der Dosierung von Azol der Formel (II) und Phosgen als kristalliner Niederschlag vorliegt, gelingt dies leicht und vollständig. Dann wird der gebildete Azol-Hydrochlorid-Niederschlag bei 20 bis 100°C, bevorzugt bei 40 bis 80°C, durch Filtration abgetrennt. Auch diese Filtration gelingt aufgrund der kristallinen Konsistenz des Niederschlages in kurzen Filtrationszeiten gut. Das N,N'-Carbonyldiazol kann aus der bei der Azol-Hydrochlorid-Abtrennung erhaltenen Mutterlauge isolieren werden, indem man die Mutterlauge auf +40 bis -70°C, bevorzugt auf +25 bis -20°C abkühlt und das dabei auskristallisierende Produkt abfiltriert. Das Produkt fällt so in gut kristallisierter Form in Reinheiten von mindestens 90%, bevorzugt mindestens 95 % an.

Ebenso ist es möglich, die Mutterlauge nach der Azolhydrochlorid-Abtrennung vollständig einzuengen und damit vom Lösungsmittel zu befreien. Das so erhaltene N,N'-Carbonyldiazol besitzt ebenfalls eine Reinheit von mindestens 90 %, bevorzugt von mindestens 95 %.

Die Hälfte des im erfindungsgemäßen Verfahren eingesetzten Azols fällt als Azol-Hydrochlorid an. Dieses kann wieder in das freie Azol überführt und somit in die Reaktion recycliert werden. Auf diesem Weg ist es möglich, eine Verdoppelung der Ausbeute an N,N'-Carbonyldiazol, bezogen auf das eingesetzte Azol, zu erreichen.

Die Wiedergewinnung von Azolen aus Azolhydrochloriden kann gemäß DE-A-198 33 913 beispielsweise so durchgeführt werden, dass man die bei der Synthese der N,N'-Carbonyldiazole entstandenen Azolhydrochloride mit einer Verbindung der Formel (III)

M(OR⁴)ₙ (III),

in der
- n: der Wertigkeit von M entspricht,
- M: für ein Alkali- oder Erdalkalimetall steht und
- R⁴: für Wasserstoff oder C₁-C₄-Alkyl steht,
umsetzt.

Diese Umsetzung erfolgt in einem Lösungsmittelgemisch aus einerseits einem aromatischen Lösungsmittel wie beispielsweise Benzol, Toluol, einem Xylol, Monochlorbenzol, einem Dichlorbenzol, einem Trichlorbenzol oder Gemischen davon, und andererseits einem Lösungsmittel der Formel

R⁴OH (IV),

in der
- R⁴: die bei Formel (III) angegebene Bedeutung hat,

In den Formeln (III) und (IV) steht R⁴ vorzugsweise für Wasserstoff oder Methyl, in Formel (III) steht M vorzugsweise für Lithium, Natrium oder Kalium.

Es ist vorteilhaft, nach der Umsetzung des Azol-Hydrochlorids mit der Verbindung der Formel (III) die gesamte Verbindung der Formel (IV), auch die bei der Reaktion von Azol-Hydrochlorid und der Verbindung der Formel (III) entstandene Verbindung der Formel (IV) abzudestillieren, das entstandene Salz MClₙ bei normaler oder erhöhter Temperatur abzufiltrieren und das gewonnene Azol nach Abtrennung des aromatischen Lösungsmittels zur erfindungsgemäßen N,N'-Carbonyl-diazol-Synthese einzusetzen.

Diese Vorgehensweise gelingt besonders gut, wenn man als Verbindung der Formel (III) LiOH, NaOH oder KOH in einem Lösungsmittelgemisch aus Wasser (das ist eine Verbindung der Formel (IV) mit R⁴ = Wasserstoff) und Chlorbenzol, Toluol, Xylol oder 2-Methyltetrahydrofuran einsetzt und das Wasser durch Azeotrop-Destillation entfernt, z.B. indem man es an einem Wasserabscheider abtrennt, oder aber, wenn man als Verbindung der Formel (III) Natriummethylat in einem Lösungsmittelgemisch aus Methanol einerseits und Chlorbenzol oder Xylol andererseits einsetzt und das Methanol durch Destillation abtrennt, z.B. indem man es aus dem Gemisch über eine wirksame Kolonne abdestilliert.

Zusammenfassend lässt sich mit dem erfindungsgemäßen Verfahren durch die Paralleidosierung von Azol und Phosgen unter Einsatz eines polaren Lösungsmittels aus der Gruppe der Ether, Ketone und chlorierten aliphatischen Lösungsmittel das als Nebenprodukt anfallende Azol-Hydrochlorid zuverlässig in einer nicht klebrigen Konsistenz erzeugen. Hierdurch können die Rühreigenschaften der Reaktionslösung verbessert und damit höhere Konzentrationen an Reaktanden und entsprechend höhere Raum-Zeit-Ausbeuten erzielt werden. Gleichzeitig wird die leichte Entfernung des Azol-Hydrochlorids aus dem Reaktionsgefäß sichergestellt, und Beschädigungen durch verhärtete Azolhydrochloride werden ausgeschlossen. Auch die Filtrationszeiten sind aufgrund des verbesserten Filtrationsverhaltens des Azolhydrochlorids überraschend kurz. Ein weiterer Vorteil der erfindungsgemäßen Verwendung eines polaren Lösungsmittels ist die sehr geringe Empfindlichkeit des Reaktionssystems bezüglich eventuell auftretender Phosgenüberschüsse während oder am Ende der Reaktion: Gegenüber Verfahren des Standes der Technik wird sowohl die Konsistenz des kristallinen Azol-Hydrochlorid Niederschlags als auch die Farbe des aus der Reaktionsmischung gewonnenen N,N-Carbonyldiazols durch geringe Phosgenüberschüsse nicht oder nur minimal beeinflusst.

### Beispiele

### Beispiel 1 (erfindungsgemäß)

In einem Kolben werden 120 g trockenes THF vorgelegt, das auf 60°C aufgeheizt wird. Von einer auf 62°C erhitzten Lösung aus 250 g Imidazol in 165 g trockenem THF werden 33 ml in das Reaktionsgefäß dosiert. Danach werden bei 60°C die restliche Menge der zuvor genannten Lösung gleichmäßig in 1,75 h mit einer Geschwindigkeit von 216 g/h und simultan dazu 96 g Phosgen mit einer Geschwindigkeit von 55 g/h in das Reaktionssystem eindosiert.

Anschließend wird die Zuleitung der Imidazol-Lösung mit 40 g THF gespült, wobei die Spüllösung ebenfalls in den Reaktionskolben überführt wird. Es wird 1,5 h bei 60°C nachgerührt. Um eine phosgenfreie Reaktionsmischung sicherzustellen, werden bei 750 mbar und 55°C 19 g Lösungsmittel abdestilliert und verworfen. Das als Nebenprodukt entstehende Imidazolhydrochlorid (isoliertes Trockengewicht: 209 g) wird durch Filtration bei 60°C entfernt, wobei der Filterkuchen mit 100 g auf 60°C erhitztem THF nachgewaschen wird. Aus den vereinigten Filtraten wird das Lösungsmittel bei 45°C und 20 mbar entfernt. Es werden 130 g Carbonylbisimidazol einer Reinheit von 98,2 %, entsprechend einer Ausbeute von 86 % der Theorie erhalten.

### Beispiel 2 (Vergleich)

In einen Kolben werden 250 g Imidazol in 285 g trockenem THF vorgelegt. Die Mischung wird auf 60°C geheizt. Bei dieser Temperatur werden 92 g Phosgen mit einer Geschwindigkeit von 55 g/h in den Kolben eindosiert. Man erhält an diesem Punkt eine so dicke Suspension, dass nur noch Teile des Reaktionsgemisches von dem Rührer durchmischt werden. Der Versuch muss daher an dieser Stelle abgebrochen und der Inhalt des Kolbens verworfen werden.

### Beispiel 3 (Vergleich)

In einem Kolben werden 218 g trockenes Aceton vorgelegt, das auf 45°C aufgeheizt wird. Von einer auf 47°C erhitzten Lösung aus 125 g Imidazol in 120 g trockenem Aceton werden 20 ml in das Reaktionsgefäß dosiert. Danach werden bei 45°C die restliche Menge der zuvor genannten Imidazol-Lösung gleichmäßig in 1,75 h mit einer Geschwindigkeit von 132 g /h und simultan dazu 48 g Phosgen mit einer Geschwindigkeit von 28 g/h in den Kolben eindosiert. Anschließend wird die Zuleitung der Imidazol-Lösung mit 30 g Aceton gespült, wobei die Spüllösung ebenfalls in den Reaktionskolben überführt wird. Es wird 1,5 h bei 45°C nachgerührt.

Um eine phosgenfreie Reaktionsmischung sicherzustellen, werden bei 770 mbar und 45°C 19 g Lösungsmittel abdestilliert und verworfen.

Das als Nebenprodukt entstehende Imidazolhydrochlorid (isoliertes Trockengewicht: 102 g) wird durch Filtration bei 45°C entfernt, wobei der Filterkuchen mit 50 g auf 45°C aufgeheiztem Aceton nachgewaschen wird.

Die vereinigten Filtrate werden auf 0°C gekühlt und 30 min bei dieser Temperatur nachgerührt. Das ausfallende Carbonylbisimidazol wird durch Filtration abgetrennt, wobei der Niederschlag nochmals mit 30 g auf 0°C gekühltem Aceton verdrängt wird.

Eine Trocknung des Niederschlags bei 45°C und 20 mbar liefert 53,7 g Carbonylbisimidazol einer Reinheit von 97,5 % entsprechend einer Ausbeute von 71 % der Theorie.

### Beispiel 4 (erfindungsgemäß)

In einem Kolben werden 120 g trockenes THF vorgelegt, das auf 60°C aufgeheizt ist. Von einer auf 62°C erhitzten Lösung aus 250 g Imidazol in 165 g trockenem THF, werden 8 ml in das Reaktionsgefäß dosiert. Danach wird bei 60°C die restliche Menge der zuvor genannten Imidazol Lösung gleichmäßig in 1,75 h mit einer Geschwindigkeit von 234 g /h und simultan dazu 91 g Phosgen mit einer Geschwindigkeit von 52 g/h in den Kolben eindosiert. Anschließend wird die Zuleitung der Imidazol-Lösung mit 40 g THF gespült, wobei die Spüllösung ebenfalls in den Reaktionskolben überführt wird. Es wird 1,5 h bei 60°C nachgerührt.

Um eine phosgenfreie Reaktionsmischung sicherzustellen, wird bei 750 mbar und 55°C 17 g Lösungsmittel abdestilliert und verworfen.

Das als Nebenprodukt entstehende Imidazolhydrochlorid (isoliertes Trockengewicht: 185 g) wird durch Filtration bei 60°C entfernt, wobei der Filterkuchen mit 100 g auf 60°C aufgeheiztem THF nachgewaschen wird.

Die vereinigten Filtrate werden auf 0°C gekühlt und 30 min bei dieser Temperatur nachgerührt. Das ausfallende Carbonylbisimidazol wird durch Filtration abgetrennt, wobei der Niederschlag nochmals mit 50 g auf 0°C gekühltem THF verdrängt wird.

Eine Trocknung des Niederschlags bei 45°C und 20 mbar liefert 107 g Carbonylbisimidazol einer Reinheit von 92,8 % entsprechend 67 % der Theorie.

## Patentansprüche

1. Verfahren zur Herstellung von N,N'-Carbonyldiazolen der allgemeinen Formel (I) 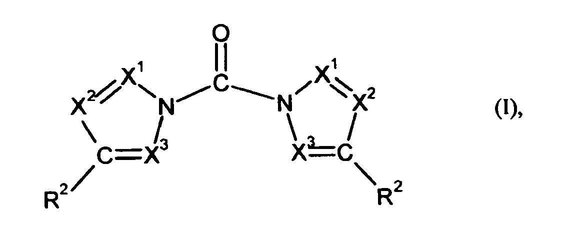 wobei entweder
X¹ und X³ unabhängig voneinander jeweils für CR¹ stehen, wobei R¹ Wasserstoff oder geradkettiges oder verzweigtes C₁-C₆-Alkyl bedeutet und
X² für Stickstoff steht und
R² Wasserstoff bedeutet,
oder
X¹ und X³ für CR¹ stehen, wobei der in X¹ befindliche Rest R¹ Wasserstoff oder geradkettiges oder verzweigtes C₁-C₆-Alkyl bedeutet und der in X³ befindliche Rest R¹ gemeinsam mit R² eine -CH=CH-CH=CH-Brücke bildet, und
X² für Stickstoff steht,
durch Umsetzung von Azolen der allgemeinen Formel (II), 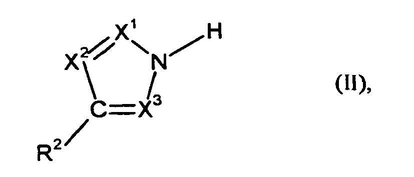 in der die verwendeten Reste und Symbole die für die allgemeine Formel (I) angegebenen Bedeutungen haben,
mit Phosgen in einem polaren Lösungsmittel, welches **dadurch gekennzeichnet ist, dass**
(i) ein polares Lösungsmittel aus der Gruppe bestehend aus Ethern, Ketonen und chlorierten aliphatischen Lösungsmitteln verwendet wird, welches einen Wassergehalt von maximal 0,5 Gew.% besitzt, und
(ii) dass man Azol der allgemeinen Formel (II) sowie das Phosgen in dieses Lösungsmittel so eindosiert, dass in der Zeit, in der 1 mol Azol der allgemeinen Formel (II) eindosiert wird, gleichzeitig 0,17 bis 0,34 mol Phosgen eindosiert werden, wobei die Reaktandenkonzentration (Azolkonzentration) bei über 40 Gew.% liegt.

2. Verfahren nach Anspruch 1, **dadurch gekennzeichnet, dass** entweder zwei verschiedene oder aber auch nur ein einziges Azol der allgemeinen Formel (II) eingesetzt werden.

3. Verfahren nach einem oder mehreren der Ansprüche 1 bis 2, **dadurch gekennzeichnet, dass** ein oder zwei Azole der allgemeinen Formel (II) eingesetzt wird, bei denen unabhängig voneinander X¹ für CH, X² für Stickstoff und X³ für CR¹ stehen, wobei R¹ und R² gemeinsam eine -CH=CH-CH=CH-Brücke bilden.

4. Verfahren nach einem oder mehreren der Ansprüche 1 bis 3, **dadurch gekennzeichnet, dass** Imidazol oder Benzimidazol, als Azol der allgemeinen Formel (II) eingesetzt wird.

5. Verfahren nach einem oder mehreren der Ansprüche 1 bis 4, **dadurch gekennzeichnet, dass** insgesamt pro mol Azol der allgemeinen Formel (II) 0,2 bis 0,3 mol eingesetzt werden.

6. Verfahren nach einem oder mehreren der Ansprüche 1 bis 5, **dadurch gekennzeichnet, dass** als Ether MTBE, Dimethylether, Diethylether, Dibutylether, THF, 2-Methyl-THF, 2,5-Dimethyl-THF, Dioxan, Ethylenglycoldimethylether oder Ethylenglycoldiethylether, Anisol und chlorierte Derivate des Anisols zum Einsatz sowie Gemische der vorgenannten Lösungsmittel eingesetzt werden.

7. Verfahren nach einem oder mehreren der Ansprüche 1 bis 5, **dadurch gekennzeichnet, dass** als Ketone Aceton, 2-Butanon, Diethylketon, Dipropylketon, Cyclopentanon, Cyclohexanon oder Cycloheptanon, sowie Gemische der vorgenannten Lösungsmittel eingesetzt werden.

8. Verfahren nach einem oder mehreren der Ansprüche 1 bis 5, **dadurch gekennzeichnet, dass** als chlorierte aliphatische Lösungsmittel Methylenchlorid, Chloroform oder 1,2-Dichlorethan eingesetzt werden.

9. Verfahren nach einem oder mehreren der Ansprüche 1 bis 8, **dadurch gekennzeichnet, dass** das polare Lösungsmittel einen Wassergehalt von maximal 0,2 Gew.-%, besitzt.

10. Verfahren nach einem oder mehreren der Ansprüche 1 bis 9, **dadurch gekennzeichnet, dass** man das Azol der allgemeinen Formel (II) sowie das Phosgen simultan zu dem polaren Lösungsmittel aus der Gruppe bestehend aus Ethern, Ketonen und chlorierten aliphatischen Lösungsmitteln so zudosiert, dass in der Zeit, in der 1 mol Azol der allgemeinen Formel (II) eindosiert wird, gleichzeitig 0,2 bis 0,3 mol, mol Phosgen eindosiert werden.

11. Verfahren nach einem oder mehreren der Ansprüche 1 bis 10, **dadurch gekennzeichnet, dass** man im Reaktionsgefäß bis zu 10 Gew.-%, der Gesamtmenge des Azols der allgemeinen Formel (II) in Form einer Lösung oder Suspension im Reaktionsgefäß vorlegt und anschließend die weitere Menge des Azols und das Phosgen simultan wie in Anspruch 1 und 10 angegeben zudosiert.

## Claims

1. Process for preparing N,N'-carbonyldiazoles of the general formula (I) 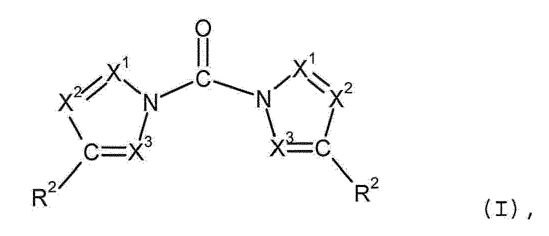 where either
X¹ and X³ independently of one another are each CR¹, R¹ being hydrogen or straight-chain or branched C₁-C₆ alkyl, and
X² is nitrogen and
R² is hydrogen,
or
X¹ and X³ are CR¹, the radical R¹ in X¹ being hydrogen or straight-chain or branched C₁-C₆ alkyl and the radical R¹ in X³ forming, together with R², a -CH=CH-CH=CH- bridge, and
X² is nitrogen,
by reacting azoles of the general formula (II), 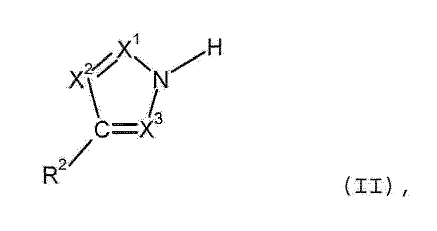 in which the radicals and symbols used have the definitions indicated for the general formula (I),
with phosgene in a polar solvent, which process is **characterized in that**
(i) a polar solvent from the group consisting of ethers, ketones and chlorinated aliphatic solvents is used which possessed a maximum water content of 0.5% by weight, and
(ii) **in that** the azole of the general formula (II) and also the phosgene are metered into this solvent in such a way that in the time within which 1 mol of azole of the general formula (II) is metered in, at the same time 0.17 to 0.34 mole of phosgene is metered in, the reactant concentration (Azole concentration) being more than 40% by weight.

2. Process according to Claim 1, **characterized in that** either two different azoles or else only one single Azole of the general formula (II) are or is used.

3. Process according to one or more of Claims 1 to 2, **characterized in that** one or two azoles of the general formula (II) is or are used in which independently of one another X¹ is CH, X² is nitrogen and X³ is CR¹, R¹ and R² together forming a -CH=CH-CH=CH- bridge.

4. Process according to one or more of Claims 1 to 3, **characterized in that** imidazole or benzimidazole is used as the azole of the general formula (II).

5. Process according to one or more of Claims 1 to 4, **characterized in that** in total 0.2 to 0.3 mole of phosgene is used per mole of Azole of the general formula (II).

6. Process according to one or more of Claims 1 to 5, **characterized in that** ethers used are MTBE, dimethyl ether, diethyl ether, dibutyl ether, THF, 2-methyl-THF, 2,5-dimethyl-THF, dioxane, ethylene glycol dimethyl ether or ethylene glycol diethyl ether, anisole and chlorinated derivatives of anisole, and also mixtures of the aforementioned solvents.

7. Process according to one of more of Claims 1 to 5, **characterized in that** ketones used are acetone, 2-butanone, diethyl ketone, dipropyl ketone, cyclopentanone, cyclohexanone or cycloheptanone, and also mixtures of the aforementioned solvents.

8. Process according to one or more of Claims 1 to 5, **characterized in that** chlorinated aliphatic solvents used are methylene chloride, Chloroform or 1,2-dichloroethane.

9. Process according to one or more of Claims 1 to 8, **characterized in that** the polar solvent possesses a water content of not more than 0.2% by weight.

10. Process according to one or more of Claims 1 to 9, **characterized in that** the azole of the general formula (II) and also the phosgene are metered in simultaneously to the polar solvent from the group consisting of ethers, ketones and chlorinated aliphatic solvents is such a way that in the time within which 1 mol of azole of the general formule (II) is metered in, at the same time 0.2 to 0.3 mole of phosgene is metered in.

11. Process according to one or more of Claims 1 to 10, **characterized in that** the reaction vessel is charged with up to 10% by weight of the total amount of the Azole of the general formula (II), in the form of a solution or suspension, and subsequently the further amount of the azole, and the phosgene, are metered in simultaneously as specified in Claims 1 and 10.

## Revendications

1. Procédé pour la préparation de N,N'-carbonyldiazoles de formule générale (I) 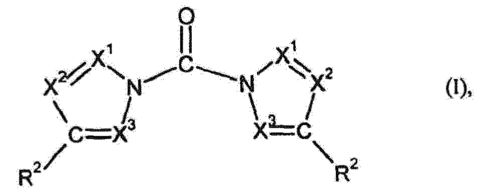 où soit
X¹ et X³ représentent, indépendamment l'un de l'autre, à chaque fois CR¹, R¹ signifiant hydrogène ou C₁-C₆-alkyle linéaire ou ramifié et
X² représente azote, et
R² signifie hydrogène, soit
X¹ et X³ représentant CR¹, le radical R¹ se trouvant dans X¹ signifiant hydrogène ou C₁-C₆-alkyle linéaire ou ramifié et le radical R¹ se trouvant dans X³ formant, ensemble avec R², un pont -CH=CH-CH=CH-, et
X² représente azote,
par transformation d'azoles de formule générale (II), 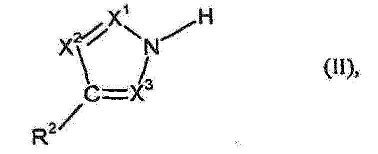 dans laquelle les radicaux et symboles utilisées présentent les significations indiquées pour la formule générale (I),
avec du phosgène dans un solvant polaire, qui est **caractérisé en ce que**
(i) un solvant polaire du groupe constitué par les éthers, les cétones et les solvants aliphatiques chlorés, est utilité, qui présente une teneur en eau d'au maximum 0,5% en poids, et
(ii) on dose l'azole de formule générale (II) ainsi que le phosgène dans ce solvant de manière telle que pendant le temps durant lequel 1 mole d'azole de formule générale (II) est dosée, 0,17 à 0,34 mole de phosgène est dosée simultanément, la concentration en réactifs (concentration en gazole) étant supérieure à 40% en poids.

2. Procédé selon la revendication 1, **caractérisé en ce qu'**on utilise, soit deux gazoles différents, soit également seulement un azole unique de formule générale (II).

3. Procédé selon l'une ou plusieurs des revendications 1 à 2, **caractérisé en ce qu'**on utilisé un ou deux azoles de formule générale (ici), dans laquelle, indépendamment les uns des autres, X¹ représente CH, X² représente azote et X³ représente CR¹, R¹ et R² formant ensemble un pont -CH=CH-CH=CH-.

4. Procédé selon l'une ou plusieurs des revendications 1 à 3, **caractérisé en ce qu'**on utilise de l'imidazole ou du benzimidazole, comme azole de formule générale (II).

5. Procédé selon l'une ou plusieurs des revendications 1 à 4, **caractérisé en ce qu'**on utilise au total, par mole d'azole de formule générale (II), 0,2 à 0,3 mole de phosgène.

6. Procédé selon l'une ou plusieurs des revendications 1 à 5, **caractérisé en ce qu'**on utilise, comme éthers, le MTBE, le diméthyléther, le diéthyléther, le dibutyléther, le THF, le 2-méthyl-THF, le 2,5-diméthyl-THF, le dioxane, l'éthylèneglycoldiméthyléther ou l' éthylèneglycoldiéthyléther l'anisol et les dérivés chlorés de l'anisol, ainsi que des mélanges des solvants susmentionnés.

7. Procédé selon l'une ou plusieurs des revendications 1 à 5, **caractérisé en ce qu'**on utilise, comme cétones, l'acétone, la 2-butanone, la diéthylcétone, la dipropylcétone, la cyclopentanone, la cyclohexanone ou la cycloheptanone, ainsi que des mélanges des solvants susmentionnés.

8. Procédé selon l'une ou plusieurs des revendications 1 à 5, **caractérisé en ce qu'**on utilise, comme solvants aliphatiques chlorés, le chlorure de méthylène, le chloroforme ou le 1,2-dichloroéthane.

9. Procédé selon l'une ou plusieurs des revendications 1 à 8, **caractérisé en ce que** le solvant polaire présente une teneur en eau d'au maximum 0,2% en poids.

10. Procédé selon l'une ou plusieurs des revendications 1 à 9, **caractérisé en ce qu'**on dose l'azole de formule générale (II) ainsi que le phosgène simultanément dans le solvant polaire du groupe constitué par les éthers, les cétones et les solvants aliphatiques chlorés, de manière telle que pendant le laps de temps durant lequel 1 mole d'azole de formule générale (II) est dosée, 0,2 à 0,3 mole de phosgène est dosée simultanément.

11. Procédé selon l'une ou plusieurs des revendications 1 à 10, **caractérisé en ce qu'**on dispose au préalable dans le récipient de réaction jusqu'à 10% en poids de la quantité totale de l'azole de formule générale (II) sous forme d'une solution ou d'une suspension puis on y dose la quantité résiduelle de l'azole et le phosgène simultanément, comme indiqué dans la revendication 1 et 10.
